(19) Europäisches Patentamt
European Patent Office
Office européen des brevets

(11) Publication number: 0 004 408
B1

# (12) EUROPEAN PATENT SPECIFICATION

(45) Date of publication of patent specification: 11.11.81

(21) Application number: 79200140.6

(22) Date of filing: 22.03.79

(51) Int. Cl.³: C 07 C 2/40, C 07 C 11/12, B 01 J 31/24, B 01 J 37/16, B 01 J 37/18, B 01 J 31/28

## (54) A process for preparing 1,7-octadiene and for activating a catalyst suitable for use in the process.

(30) Priority: 27.03.78 US 890114

(43) Date of publication of application:
03.10.79 Bulletin 79/20

(45) Publication of the grant of the European patent:
11.11.81 Bulletin 81/45

(84) Designated Contracting States:
DE FR GB NL

(56) References cited:
GB - A - 1 341 324
US - A - 3 732 328
US - A - 3 823 199

JOURNAL OF ORGANOMETALLIC CHEMISTRY, volume 55, July 16 1973, LAUSANNE (CH)
P. ROFFIA et al.: "Catalysis by Palladium Salts IV. Selective Hydrogenation with Formic Acid in the Palladium Catalysed Dimerisation of 1,3-butadiene; Syntheses of 1,7-octadiene", pages 405—407

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Nozaki, Kenzie
1407 Lakecliff
Houston Texas 77077 (US)

(74) Representative: Keuzenkamp, Abraham et al.
c/o Shell Internationale Research Maatschappij B.V. P.O. Box 302
NL-2501 CH 's-Gravenhage (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 004 408 B1

Courier Press, Leamington Spa, England.

# A process for preparing 1,7-octadiene and for activating a catalyst suitable for use in the process.

The present invention is concerned with a process for preparing 1,7-octadiene by hydrodimerizing butadiene in the presence of palladium-phosphine complex catalysts.

Linear dimerization of butadiene (1,3-butadiene) provides a source of $C_8$ unsaturated hydrocarbon intermediates useful for the synthesis of diacids, diesters, diols or diamines. A particularly preferred dimer is 1,7-octadiene which has terminal double bonds and allows the production of product having only terminal functional groups.

It is known to prepare mixtures of octadienes, e.g. 1,6- and 1,7-octadienes, by contacting 1,3-butadiene with palladium in the presence of a reducing agent, such as formic acid. Solvents may be present which may be non-polar solvents, e.g. see U.S. Patent Specification No. 3,823,199, or polar solvents, e.g. see U.S. Patent Specification 3,732,328, including amines e.g. see U.K. Patent Specification No. 1,341,324. Tertiary phosphines such as triphenyl phosphine may also be present e.g. see U.S. Patent Specification 3,732,328 and Journal of Organometallic Chemistry, 55 (1973) pages 405—407. Organic bases may also be present e.g. see Tetrahedron Letters No. 2, 1972 pages 163—164.

The Applicants have now surprisingly found that the activity of the palladium compound-trisorganophosphine complex catalyst is significantly enhanced if the catalyst is contacted with a reducing agent at a temperature of flow 20 to 90°C, preferably for 0.1 to 5 hours, prior to contact with the butadiene. The triethylamine salt of formic acid, hydrazine, hydrogen and carbon monoxide are particularly desirable reducing agents. The hydrodimerization of butadiene is suitably carried out in the presence of formic acid, a base and optionally a solvent.

None of the references cited above teach the concept of increasing the activity of the palladium-phosphine catalyst by reducing it at 20—90°C prior to contacting it with the butadiene.

Accordingly, the present invention is concerned with a process for preparing 1,7-octadiene by hydrodimerizing butadiene in the presence of a palladium compound trisorgano-mono-phosphine complex catalyst, characterized in that the catalyst is contacted with a reducing agent at a temperature of from 20 to 90°C prior to contacting the catalyst with the butadiene.

According to another aspect of the present invention, a process for activating a palladium compound trisorgano-mono-phosphine complex catalyst suitable for use in the hydrodimerization of butadiene to 1,7-octadiene is characterized in that the catalyst is contacted with a reducing agent at a temperature of from 20 to 90°C.

The palladium compounds used to form the catalyst utilized in this invention are those compounds of palladium which readily form complexes with trisorganophosphines. These may be organic or inorganic compounds.

Suitable palladium compounds include the palladium carboxylates, particularly palladium carboxylates derived from alkanoic acids containing up to six carbon atoms such as palladium acetate (OAC), complexes such as palladium acetylacetonate (AcAc), bisbenzonitrile palladium (II) and lithium palladous chloride as well as the palladium halides, nitrates and sulphates such as palladous chloride and palladium nitrate ($Pd(NO_3)_2(OH)_2$) and palladium sulphate. The palladium compound will be present in the reaction mixture in catalytic amounts; preferably from about 1 to about $10^{-6}$ molar and more preferably from $10^{-1}$ to about $10^{-4}$ molar. Preferred palladium compounds are palladium nitrate, palladium sulphate, palladium acetate or palladium acetylacetonate.

Any tertiary phosphine which can be dissolved in the reaction mixture may be used. The bisphosphines, such as 1,3-bisphenylphosphinopropane and 1,4-bisdiphenylphosphinobutane, will not function in the present invention as the tertiary phosphine, the butadiene conversions obtained are unsatisfactory. Accordingly, mono-phosphines are used. Suitable mono-phosphines are represented by the formula:

$$R_a\text{—}\underset{\displaystyle R_c}{\underset{|}{P}}\text{—}R_b$$

wherein $R_a$, $R_b$ and $R_c$ may be the same or different and are selected from aryl such as phenyl, p-tolyl, o-tolyl, m-tolyl, m-chlorophenyl, phenoxy, p-methylphenoxy, p-anisoyl, m-anisoyl and the like, alkyl of 1 to 8 carbon atoms, preferably 1 to 5 carbon atoms, alkoxy having from 1 to 8 carbon atoms, but preferably from 1 to 3 carbon atoms. Preferably, $R_a$, $R_b$ and $R_c$ represent an aryl or alkyl group, or a mixture thereof, each group having 1 to 12 carbon atoms. The more preferred tertiary phosphines of the above formula, however, are the triaryl and trialkylphosphines. The most preferred tertiary phosphines have the following general formula:

$$R_1R_2R_3P$$

wherein $R_1$ is an aralkyl or a cycloalkyl group or a branched alkyl or branched alkenyl group having from

3 to 10 carbon atoms with branching occurring at a carbon atom no more than two carbon atoms from the phosphorus atom and $R_2$ and $R_3$ are $R_1$ or independently are alkyl groups having from 1 to 10 carbon atoms, alkenyl groups having up to 10 carbon atoms or aryl groups having up to 10 carbon atoms.

By branched is meant that the $\alpha$- and $\beta$-carbon atoms, relative to the phosphorus atom, of the alkyl group may not both be $-CH_2-$ linkages. Hydrocarbyl groups are preferred.

Illustrative of the $R_1$ moiety are, for branched alkyl, isopropyl, sec-butyl, tert-butyl, isobutyl, neopentyl, sec-pentyl, tert-pentyl, 2-methylbutyl, sec-hexyl, tert-hexyl, 2,2-dimethylpropyl; for aralkyl, benzyl, alpha-methylbenzyl, alpha, alpha-dimethylbenzyl, alpha-methyl-alpha-ethylbenzyl, phenylethyl, phenylisopropyl, phenyl-tert-butyl; for branched alkenyl, crotyl, methallyl, 1-methyl-ethenyl, 1-methyl-2-propenyl, 2-methyl-2-propenyl, 1,1-dimethyl-2-propenyl, 1-methyl-3-butenyl and, for cycloalkyl, cyclopropyl, cyclobutyl, cyclopentyl, cyclohexyl, cycloheptyl, and the like.

Illustrative of $R_2$ and $R_3$ radicals are, for example, methyl, ethyl, propyl, butyl, pentyl, hexyl, octyl, nonyl and decyl for alkyl; allyl, crotyl and methallyl for alkenyl, and phenyl, tolyl, xylyl, ethylphenyl, propylphenyl for aryl. The preferred phosphine is tri(isopropyl)phosphine. Two or more of the instant phosphines may be used in the same reaction. The mole ratio of tertiary phosphine to palladium compound is preferably at least 1, more preferably ranges from 1:1 to 20:1 and particularly from 2:1 to 5:1. The use of the phosphines of the invention provides extremely high selectivities to 1,7-octadiene.

Alternatively, the palladium compound and tertiary phosphine may be bound onto a cross-linked synthetic resin instead of being dissolved in the reaction medium. Acceptable cross-linked synthetic resins include cross-linked polystyrene, poly(alpha-alkyl) acrylates, polycarbonates, polyamides and the like.

The bound tertiary phosphine may have the general formula:

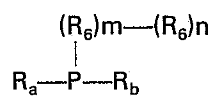

wherein $R_a$ and $R_b$ are defined previously, and $R_6$ represents the repeating unit of the synthetic resin and where m is a positive integer, n is 0 or a positive integer, m + n equal the total number of repeating units in resin and the percentage of the repeating units substituted with the tertiary phosphine is represented by the formula:

$$\frac{m}{m+n} \times 100\%$$

The number of repeating units substituted with the tertiary phosphine is not critical. When less than 5% of the repeating units contain a phosphine substituent, large quantities of the resin must be used to form the bound catalyst. Accordingly, it is desirable to have at least 10% of the repeating units substituted with a tertiary phosphine. It is preferred, however, that from 20 to 40% of the repeating units contain a phosphine substituent. The substituent can be introduced into the resin using well-known techniques, such as those described in the Journal of the American Chemical Society, *97* (7) 1749 (1975) and in Ann. N.Y. Academy of Sciences, *239*, 76 (1974). In accordance with those techniques, the palladium compound is complexed with the phosphine-substituted resin by admixing in a solvent for palladium acetate.

The palladium compound trisorgano phosphine complexes are typically prepared by reacting the tertiary phosphine with the appropriate palladium compound as, for example, represented by the following equations:

$$2R_3P + (PhCN)_2PdCl_2 \rightarrow (R_3P)_2PdCl_2$$

$$(R_3P)_2PdCl_2 + Ag_2CO_3 \rightarrow (R_3P)_2PdCO_3$$

$$(R_3P)_2PdO_2 + SO_2 \rightarrow (R_3P)_2PdSO_4$$

$$(R_3P)_2PdO_2 + N_2O_4 \rightarrow (R_3P)_2Pd(NO_3)_2$$

or may be made in situ by adding the palladium compound and the phosphine directly to the reactor.

The catalyst pretreatment of this invention comprises contacting a palladium compound trisorganophosphine complex prepared as described above with a reducing agent at a temperature of from about 20 to about 90°C, preferably for from about 0.1 to about 5 hours. Time is not the critical limitation as is temperature. Longer times are operable, although they become uneconomic. The reducing agent may be gaseous, solid or liquid. Examples of gaseous agents are hydrogen, and carbon monoxide. Examples of liquid or solid reducing agents are hydrazine, $NaBH_4$, $NaOCH_3$, trialkyl

phosphines e.g. $(isopropyl)_3P$, Cu, Na, Al alkyls, etc. A particularly preferred agent is the trialkylamine or ammonium salt of formic acid which is utilized in the hydrodimerization reaction.

The reduction may be carried out in a separate autoclave or preferably is carried out in the hydrodimerization reactor prior to the introduction of the butadiene. The palladium compound-trisorganophosphine complex may be dissolved in a solvent prior to reduction. The preferred solvents are those used in the hydrodimerization reaction described below.

Solvents are not essential to the hydrodimerization reaction, but a good organic solvent can promote the rate of reaction by a factor of two or more.

The non-polar solvents disclosed in U.S. Patent Specification No. 3,823,199 such as paraffinic, cycloparaffinic or aromatic solvents are useful in the process of this invention. The solvent can be paraffin or cycloparaffin containing 5 to 16 carbon atoms, such as hexane, dodecane, pentadecane, cyclohexane, methylcyclohexane and the like. Suitable solvents also include aromatic hydrocarbons such as benzene, lower alkyl substituted aromatic hydrocarbons such as toluene, m-, p- and o-xylene, halogenated aromatic hydrocarbons including chloro, bromo and iodo substituted, such as chloro benzene and the like. Halogenated lower aliphatic compounds such as chloroform, methylene chloride, carbon tetrachloride and the like may be used, in particularly chloroform is preferred.

Further useful solvents are the amine solvents disclosed in U.K. Patent Specification No. 1,341,324. A wide range of amines are useful provided that they are liquid under reaction conditions. Tertiary amines are preferred to primary and secondary amines. Suitable amine solvents include alkylamines, cycloalkylamines, arylamines and heterocyclic amines such as morpholine, pyridine, piperazine and piperidine. Examples of these classes of amines are the lower alkylamines containing 2 to 6 carbon atoms in each alkyl group such as triethylamine; monocyclohexylamine, and N-alkyl-cyclohexylamines containing up to 12 carbon atoms. The preferred amine solvent is pyridine.

Further useful solvents are those of moderate coordinating ability, and which include nitriles such as lower alkyl nitriles, hydrocarbon aromatic nitriles including acetonitrile, benzonitrile and the like, amides including benzamide, acetamide, mono- and di-substituted amides where the substituent is preferably lower alkyl. Suitable substituted amides include N-methyl acetamide, N,N-dimethyl acetamide and dimethylformamide. Dialkyl sulphoxides such as dimethyl sulphoxide and sulphones such as sulfolane and alkyl-substituted sulfolane are satisfactory. Simple ethers such as the dilower alkyl ethers including dimethyl ether, diethylether, and the like function satisfactorily. Hydrocarbon aromatic ethers such as the lower alkyl phenyl ethers may also be used, and include methyl phenyl ether (anisole), ethyl phenyl ether (phenetole) and the like. Cyclic, saturated hydrocarbon ethers such as tetrahydrofuran, tetrahydropyran and the like are also suitable solvents. Lower alkyl diethers such as dimethoxy ethane, and the like may be used. In addition, the cyclic diethers such as 1,4-dioxane are also suitable solvents.

Simple lower alkyl esters of lower alkanoic acids such as ethyl acetate, methyl acetate, methyl butyrate and the like as well as cyclic diesters such as ethylene carbonate and propylene carbonate are also suitable solvents of moderate coordinating ability. Ketones, including lower aliphatic ketones such as methyl ethyl ketone and hydrocarbon aromatic ketones such as acetophenone are also satisfactory solvents. Lower mono- and di-alkanols such as isopropanol, ethylene glycol and the like may be used if desired. The preferred solvents of moderate coordinating ability include nitriles, formamides, such as dimethylformamide, dilower alkyl ethers, lower alkyl phenyl ethers, simple lower alkyl esters of lower alkanoic acids, ketones and lower alkanols.

Particularly useful solvents include pyridine, benzene, dimethylformamide, chlorobenzene, anisole, N,N-dimethylacetamide, nitromethane, ethyl acetate, isopropanol, benzonitrile, chloroform, methyl ethyl ketone, acetonitrile, diethylether, acetophenone, toluene, ethylene glycol, ethylene carbonate, propylene carbonate and sulfolane. Particularly desired solvents are pyridine, nitromethane, ethylene carbonate and propylene carbonate.

The preferred organic solvents will have from 1 to 20 carbon atoms per molecule. Particularly desired solvents are those which give two-phase systems which allow easy solvent separation such as, for example, nitromethane, ethylene carbonate and propylene carbonate.

The amount of solvent added should be sufficient to dissolve the palladium compound-tertiary phosphine complex.

The source of hydrogen for the process is suitably formic acid. It is present in the reaction mixture as a salt of the base promoter utilized. It is thought that disassociation of the formic acid-base salt provides a suitable amount of formic acid necessary to provide the required hydrogen. Excess free acid present in the reaction mixture has an inhibitory effect on the reaction.

It is desirable that some formic acid, as the salt, be present during the entire course of the reaction. When operating the process batch-wise, this can be accomplished by adding a stoichiometric amount of formic acid initially, 1 mole of formic acid for every 2 moles of butadiene, or by continuously or periodically adding additional amounts of formic acid. It is highly desirable, however, that the ratio of base to formic acid present in the reaction medium never be less than 1.

The base must be one which can neutralize formic acid according to the reaction:

$$HCOOH + B \rightarrow HCOO^{-}HB^{+}.$$

The base may be either insoluble or soluble in the reaction medium.

The base may be organic or inorganic. Suitable organic bases typically have dissociation constants greater than $10^{-8}$ and include tertiary amines such as triethylamine, tributyl amine, dimethyl ethyl amine, lutidine, tripropyl amine, N-methyl morpholine, isoquinoline, N-methyl-2,2,6,6-tetramethyl piperidine, 2,8-(dimethylamine) naphthalene and the like.

Suitable inorganic bases includes ammonia, the hydroxide bases such as sodium hydroxide, potassium hydroxide, calcium hydroxide; ammonium hydroxide; the carbonates and bicarbonates such as sodium carbonate, sodium bicarbonate, potassium carbonate, potassium bicarbonate, calcium carbonate and the like; the weak bases such as sodium acetate, potassium acetate, ammonium carbonate, ammonium acetate and the like. When the inorganic bases are utilized, small amounts of water may be present.

The mole ratio of base to formic acid should be at least equal to 1. When organic bases are utilized, excess base may be utilized as a solvent or the amine-base salt may be used as a solvent.

The conventional catalyst used in the process of this invention is a palladium compound complexed with a tertiary phosphine.

The addition of carbon dioxide to the reaction system has been found to increase the extent of butadiene conversion, but does not affect the selectivity. When it is desired to use carbon dioxide to increase the conversion rate, the partial pressure of the $CO_2$ in the reaction system may be from about 10 to 100 psia. Since carbon dioxide is a by-product of the process, it is possible to generate sufficient carbon dioxide *in situ* to enhance the conversion rates.

The process can be either continuous or batch. The reaction temperature of the process is not critical, however, it is preferred to maintain the reaction between about 0 to about 100°C preferably between about 20 to about 70°C. The process is conducted under a sufficient pressure to maintain liquid phase conditions at the reaction temperature. Typically the pressure is autogenous.

The process of this invention is particularly useful when applied to a butadiene/isobutene/n-butenes (BBB) stream from an oil pyrolysis unit. These BBB streams are the $C_4$ cut from a thermal cracking unit typically containing 30—40% butadiene, 20—35% isobutene and 20—30% n-butenes and many minor components.

The invention will now be illustrated by reference to the following Examples.

## Example 1

Part A: No Pretreatment

To an 80 ml glass-lined autoclave were charged $2.7 \times 10^{-5}$ moles of the palladium compound listed in column 1 of Table I, $5.4 \times 10^{-5}$ moles (isopropyl)$_3$ phosphine, 2.5 g of triethylamine-formic acid salt, 10 ml of pyridine and 2 g of 1,3-butadiene. The stirred reactor was heated to 45°C for 1 hour, cooled and the product was analyzed by gas chromatography for the amount of 1,7-octadiene present. The results are shown in column 2 in Table I.

Part B: Pretreatment with $(CH_3CH_2)_3N \cdot HCO_2H$

To an 80 ml glass-lined autoclave were charged $2.7 \times 10^{-5}$ moles of the palladium compound listed in column 1 of Table I, $5.4 \times 10^{-5}$ moles of (isopropyl)$_3$ phosphine, 2.5 g of triethylamine-formic acid salt and 10 ml of pyridine. The stirred reactor was heated to 60°C for 1 hour. The reactor was then cooled to 45°C, 2 g of butadiene were injected and heating with stirring was continued for one hour. The reactor was then cooled and the product was analyzed by gas chromatography for the amount of 1,7-octadiene present. The results are shown in column 3 of Table I.

Part C: Pretreatment with Hydrogen

To an 80 ml glass-lined autoclave were charged $2.7 \times 10^{-5}$ moles of the palladium compound listed in column 1 of Table I, $5.4 \times 10^{-5}$ moles of (isopropyl)$_3$ phosphine, and 10 ml of pyridine. The stirred reactor was heated to 60°C for one hour under 100 psig of hydrogen. The reactor was then cooled to 45°C, the hydrogen vented and 2 g of butadiene and 2.5 g of $(CH_3CH_2)_3N \cdot HCO_2H$ were injected and heating with stirring was continued for one hour. The reactor was then cooled and the product was analyzed by gas chromatography for the amount of 1,7-octadiene present. The results are shown in column 4 of Table I.

TABLE I
Butadiene Converted to 1,7-Octadiene, %

| Palladium Compound | No Pretreatment | Pretreatment with $(CH_3CH_2)_3N \cdot HCO_2H$ | Pretreatment with Hydrogen |
|---|---|---|---|
| $Pd(NO_3)_2(OH)_2$ | 33.0 | 54.1 | 94.1 |
| $Pd(OAC)_2$ | 4.8 | 61.5 | 45.4 |
| $Pd(AcAc)_2$ | 10.9 | 58.5 | 46.2 |
| $PdSO_4$ | 3.8 | 34.9 | 28.8 |
| $PdCl_2$ | 2.8 | 6.35 | 8.2 |

Example 2

To an 80 ml glass-lined autoclave were charged $2.7 \times 10^{-5}$ moles of $Pd(NO_3)_2(OH)_2$ as a 10% aqueous solution, $5.4 \times 10^{-5}$ moles of $(isopropyl)_3$ phosphine, 10 ml of pyridine and the reducing agent shown in Table II. The stirred reactor was heated at 60°C for one hour. The reactor was then cooled to 40°C, 2.5 g of $(CH_3CH_2)_3N \cdot HCO_2H$ and 2 g of butadiene were added and heating and stirring were continued for one hour. The reactor was then cooled and the product was analyzed by gas chromatography for the amount of 1,7-octadiene present. The results are shown in Table II.

TABLE II

| Pre-Treatment Additive | Amount | Butadiene Converted to 1,7-Octadiene, % |
|---|---|---|
| None | | 7.1 |
| $Et_3N \cdot HOOCH$ | 2.5 g | 32 |
| Hydrogen | 100 psig | 45 |
| Carbon Monoxide | 100 psig | 19 |
| Hydrazine | 0.01 ml | 49 |
| $NaBH_4$ | 0.01 g | 16 |
| $(Isopropyl)_3P$ | 0.016 ml | 14 |

Example 3

To an 80 ml glass-lined autoclave were charged $2.7 \times 10^{-5}$ moles of palladium as palladium acetate, $5.4 \times 10^{-5}$ moles of triisopropylphosphine and 10 ml of pyridine. The autoclave was charged with 100 psig of hydrogen and heated to the temperatures shown for various charges in Table III. To the various charges were added 2.5 g of $(CH_3CH_2)_3N \cdot HCO_2H$ and 2 g of butadiene. These were run for one hour at 40°C and the results are shown in Table III.

TABLE III

| Charge | Temperature, °C | Butadiene Converted to 1,7-Octadiene, % |
|---|---|---|
| 1 | 40 | 4.87 |
| 2 | 60 | 7.71 |
| 3 | 75 | 8.62 |
| 4 | 100 | 0.38 |

## Claims

1. A process for preparing 1,7-octadiene by hydrodimerizing butadiene in the presence of a palladium compound trisorgano-mono-phosphine complex catalyst, characterized in that the catalyst is contacted with a reducing agent at a temperature of from 20 to 90°C prior to contacting the catalyst with the butadiene.

2. A process as claimed in claim 1, characterized in that the contact time is from 0.1 to 5 hours.

3. A process as claimed in claim 1 or claim 2, characterized in that the palladium compound is palladium nitrate, palladium sulphate, palladium acetate or palladium acetylacetonate.

4. A process as claimed in any one of the preceding claims, characterized in that the trisorgano-mono-phosphine is of the general formula:

$$R_1R_2R_3P$$

wherein $R_1$ is an aralkyl or cycloalkyl group or a branched alkyl or branched alkenyl group having from 3 to 10 carbon atoms with branching occurring at a carbon atom no more than two carbon atoms from the phosphorus atom and $R_2$ and $R_3$ are $R_1$ or independently are alkyl groups having from 1 to 10 carbon atoms, alkenyl groups having up to 10 carbon atoms or aryl groups having up to 10 carbon atoms.

5. A process as claimed in claim 4, characterized in that the phosphine is tri(isopropyl)phosphine.

6. A process as claimed in any one of the preceding claims, characterized in that the reducing agent is hydrogen, carbon monoxide, a trialkylamine or ammonium salt of formic acid, hydrazine, sodium borohydride or a trialkylphosphine.

7. A process as claimed in claim 6, characterized in that the reducing agent is the triethylamine salt of formic acid.

8. A process as claimed in any one of the preceding claims, characterized in that the hydrodimerization of butadiene is carried out in the presence of formic acid, a base which can neutralize formic acid and optionally a solvent.

9. A process for activating a palladium compound trisorgano-mono-phosphine complex catalyst suitable for use in the hydrodimerization of butadiene to 1,7-octadiene, characterized in that the catalyst is contacted with a reducing agent at a temperature of from 20 to 90°C.

10. A process as claimed in claim 9, characterized in that the contact time is from 0.1 to 5 hours.

11. A process as claimed in claim 9 or claim 10, characterized in that the reducing agent is hydrogen, carbon monoxide, a trialkylamine or ammonium salt of formic acid, hydrazine, sodium borohydride or a trialkylphosphine.

12. A process as claimed in any one of claims 9 to 11, characterized in that the reducing agent is the triethylamine salt of formic acid.

## Patentansprüche

1. Verfahren zur Herstellung von 1,7-Octadien durch Hydrodimerisierung von Butadien in Gegenwart eines Komplexkatalysators aus einer Palladiumverbindung und einem Tris-(organo)-monophosphin, dadurch gekennzeichnet, dass der Katalysator mit einem Reduktionsmittel bei einer Temperatur von 20 bis 90°C kontaktiert wird, bevor er mit dem Butadien in Berührung gebracht wird.

2. Verfahren gemäss Anspruch 1, dadurch gekennzeichnet, dass die Kontaktzeit 0,1 bis 5 h beträgt.

3. Verfahren gemäss Anspruch 1 oder Anspruch 2, dadurch gekennzeichnet, dass die Palladiumverbindung Palladiumnitrat, Palladiumsulfat, Palladiumacetat oder Palladiumacetylacetonat ist.

4. Verfahren gemäss einem der vorstehenden Ansprüche 1 bis 3, dadurch gekennzeichnet, dass das Tris-(organo)-monophosphin die allgemeine Formel

$$R_1R_2R_3P$$

hat, wobei $R_1$ ein Aralkyl- oder Cycloalkylrest oder ein verzweigter Alkyl- oder verzweigter Alkenylrest mit 3 bis 10 Kohlenstoffatomen ist, wobei die Verzweigung an einem Kohlenstoffatom stattfindet, das nicht mehr als 2 Kohlenstoffatome vom Phosphoratom entfernt ist, und $R_2$ und $R_3$ der Rest $R_1$ sind oder unabhängig Alkylreste mit 1 bis 10 Kohlenstoffatomen, Alkenylreste mit bis zu 10 Kohlenstoffatomen oder Arylreste mit bis zu 10 Kohlenstoffatomen sind.

5. Verfahren gemäss Anspruch 4, dadurch gekennzeichnet, dass das Phosphin Tri(isopropyl)-phosphin ist.

6. Verfahren gemäss einem der vorstehenden Ansprüche, dadurch gekennzeichnet, dass das Reduktionsmittel Wasserstoff, Kohlenmonoxid, ein Trialkylamin- oder Ammoniumsalz der Ameisensäure, Hydrazin, Natriumborhydrid oder ein Trialkylphosphin ist.

7. Verfahren gemäss Anspruch 6, dadurch gekennzeichnet, dass das Reduktionsmittel das Triäthylaminsalz der Ameisensäure ist.

8. Verfahren gemäss einem der vorstehenden Ansprüche, dadurch gekennzeichnet, dass die Hydrodimerisierung des Butadiens in Gegenwart von Ameisensäure, einer Base, die die Ameisensäure neutralisieren kann, und gegebenenfalls eines Lösungsmittels durchgeführt wird.

9. Verfahren zur Aktivierung eines Komplexkatalysators aus einer Palladiumverbindung und einem Tris-(organo)-monophosphin, der für die Verwendung bei der Hydrodimerisierung von Butadien zur 1,7-Octadien geeignet ist, dadurch gekennzeichnet, dass der Katalysator mit einem Reduktionsmittel bei einer Temperatur von 20 bis 90°C kontaktiert wird.

10. Verfahren gemäss Anspruch 9, dadurch gekennzeichnet, dass die Kontaktzeit 0,1 bis 5 h beträgt.

11. Verfahren gemäss Anspruch 9 oder Anspruch 10, dadurch gekennzeichnet, dass das Reduktionsmittel Wasserstoff, Kohlenmonoxid, ein Trialkylamin- oder Ammoniumsalz der Ameisensäure, Hydrazin, Natriumborhydrid oder ein Trialkylphosphin ist.

12. Verfahren gemäss einem der Ansprüche 9 bis 11, dadurch gekennzeichnet, dass das Reduktionsmittel das Triäthylaminsalz der Ameisensäure ist.

**Revendications**

1. Procédé pour la préparation de 1,7-octadiène en hydrodimérisant du butadiène en présence d'un catalyseur complexe composé du palladium-trisorganomonophosphine, caractérisé en ce que le catalyseur est mis en contact avec un agent réducteur à une température comprise entre 20 et 90°C avant la mise en contact du catalyseur avec le butadiène.

2. Procédé selon la revendication 1, caractérisé en ce que la durée du contact est comprise entre 0,1 et 5 heures.

3. Procédé selon la revendication 1 ou 2, caractérisé en ce que le composé du palladium est du nitrate de palladium, du sulfate de palladium, de l'acétate de palladium ou de l'acétylacétonate de palladium.

4. Procédé selon l'une des revendications 1 à 3 précédentes, caractérisé en ce que la trisorganomonophosphine est de la formule générale:

$$R_1R_2R_3P$$

dans laquelle $R_1$ est un groupe aralcoyle ou cycloalcoyle ou un groupe alcoyle ramifié ou alcényle ramifié ayant de 3 à 10 atomes de carbone, la ramification se trouvant à position qui n'est pas placée plus loin que deux atomes de carbone de l'atome de phosphore et $R_2$ et $R_3$ sont comme $R_1$ ou sont, indépendamment, des groupes alcoyle ayant de 1 à 10 atomes de carbone, des groupes alcényle ayant jusqu'à 10 atomes de carbone ou des groupes aryle ayant jusqu'à 10 atomes de carbone.

5. Procédé selon la revendication 4, caractérisé en ce que la phosphine est la tri(isopropyl)phosphine.

6. Procédé selon l'une revendications précédentes, caractérisé en ce que l'agent réducteur est de l'hydrogène, de l'oxyde de carbone, un sel de trialcoylamine ou d'ammonium d'acide formique, l'hydrazine, le borohydrure de sodium ou une trialcoylphosphine.

7. Procédé selon la revendication 6, caractérisé en ce que l'agent réducteur est le sel de triéthylamine d'acide formique.

8. Procédé selon l'une des revendications précédentes, caractérisé en ce que l'hydrodimérisation du butadiène est conduite en présence d'acide formique, d'une base capable de neutraliser l'acide formique et éventuellement d'un solvant.

9. Procédé pour l'activation d'un catalyseur complexe composé du palladium-trisorganomonophosphine convenant à l'emploi dans l'hydrodimérisation de butadiène en 1,7-octadiène, caractérisé en ce que le catalyseur est mis en contact avec un agent réducteur à une température comprise entre 20 et 90°C.

10. Procédé selon la revendication 9, caractérisé en ce que la durée du contact est comprise entre 0,1 et 5 heures.

11. Procédé selon la revendication 9 ou 10, caractérisé en ce que l'agent réducteur est l'hydrogène, l'oxyde de carbone, un sel de trialcoylamine ou d'ammonium d'acide formique, l'hydrazine, le borohydrure de sodium ou une trialcoylphosphine.

12. Procédé selon l'une des revendications 9 à 11, caractérisé en ce que l'agent réducteur est le sel de trialcoylamine d'acide formique.